# EUROPEAN PATENT SPECIFICATION

(11) **EP 4 247 441 B1**
(45) Date of publication and mention of the grant of the patent: **19.08.2026**
(21) Application number: 21830491.3
(22) Date of filing: 18.11.2021
(51) Int. Cl.: A61L 2/20, A61L 2/24, A61L 9/015, G06N 7/00, G06N 20/00

(54) **SYSTEM FOR THE DISINFECTION OF A CLOSED ENVIRONMENT BY OZONATION**
SYSTEM ZUR DESINFEKTION EINER GESCHLOSSENEN UMGEBUNG DURCH OZONIERUNG
SYSTÈME DE DÉSINFECTION D'UN ENVIRONNEMENT FERMÉ PAR OZONISATION

(30) Priority: 18.11.2020 IT 202000027627
(43) Date of publication of application: 27.09.2023
(73) Proprietor: Degl'Innocenti, Paolo, 50131 Firenze (IT); Sabino Monteiro, Maria da Conceiçao, 50131 Firenze (IT)
(72) Inventor: Degl'Innocenti, Paolo, 50131 Firenze (IT); Sabino Monteiro, Maria da Conceiçao, 50131 Firenze (IT)
(74) Representative: ABM Agenzia Brevetti & Marchi
(86) International application number: PCT/IB2021/060711
(87) International publication number: WO 2022/107045

(56) References cited:
- JP-A- 2005 231 977
- JP-B2- H08 684

## Description

### Field of the invention

The present invention relates to the field of sanitizing environments using ozone.

### Description of the prior art

As known, thanks to the high oxidizing power, the ozone molecules, coming into contact with harmful agents (such as dust, viruses, bacteria, particulates, gases, molds, spores), are able to oxidize the molecules in a few minutes, cancelling the pathogenic effect and reducing bad odours. Furthermore, being heavier than air, ozone is able to penetrate deeply into the less accessible interstices or between the fibres of the fabrics, ensuring the abatement of the majority of pollutants.

For this reason, ozone is widely used for the disinfection of the air inside closed environments or ventilation channels, for the sanitization of furniture, walls, upholstery and floors, as well as for washing and sanitation of foods such as fruit and vegetables. JP2005231977 and JPH03150207 disclose ozone sanitizing systems.

Furthermore, once the molecules of harmful agents have been oxidized, ozone becomes pure oxygen, chemically stable and not harmful to people's health.

However, beyond certain concentration thresholds, ozone not yet transformed into oxygen can be harmful to health, producing disorders, intoxication and even death.

Since concentrations higher than tolerable thresholds are often necessary for an effective and quick sanitation of the environments, a control system is essential that allows safe management, avoiding translating the numerous advantages of ozone into possible problems for who should use it.

### Summary of the invention

It is therefore a feature of the present invention to provide a system for sanitizing a closed environment that allows to fully automate the sanitation process and the subsequent monitoring of the ozone depletion.

It is also a feature of the present invention to provide such a system which allows to obtain a concentration of ozone necessary for sanitization within a desired time.

It is still a feature of the present invention to provide such a system that allows to predict the time necessary, after sanitization, to obtain an ozone concentration lower than the potentially harmful threshold, that is the time necessary to allow the return of people inside closed environment in total safety.

It is still a feature of the present invention to provide such a system that allows to speed up the calculation of the time required for ozone removal based on sanitization carried out previously.

It is a further feature of the present invention to provide such a system that allows the exchange of information between the sanitizing devices and an artificial intelligence software located remotely, in order to increase a database containing statistical data on the sanitization carried out.

It is also a feature of the present invention to provide such a system which allows to certify the correct execution of the sanitizing operations and, possibly, to provide the relative documentation to the sanitary authorities responsible for the sanitizing controls of the environments.

These and other objects are achieved by a system for sanitizing a closed environment, said closed environment being associated with determined environmental parameters, said system comprising:
- at least one generator arranged to emit ozone in the closed environment;
- a control unit arranged to operate the generator to adjust the emission of ozone; whose main feature is that it also comprises an ozone abatement device comprising:
   - at least one capacitor adapted to generate an electric field having an intensity value greater than 30.000 V/m
   - a current sensor configured to measure an electric current intensity value passing through the capacitor;
   said ozone abatement device being configured in such a way that, when a determined amount of ozone passes through said or each capacitor, the electric field transforms the determined amount of ozone in a determined amount of oxygen releasing a plurality of electric charges, said plurality of electric charges producing a ion current in the capacitor, said ion current having an intensity proportional to the concentration *C* of ozone in the closed environment, said control unit arranged a:
   periodically acquire from the current sensor an intensity value of ion current passing through the capacitor;
   calculate, on the basis of the intensity value of ion current, a value of concentration *C* of ozone in the closed environment;
   command an emission of ozone by the generator and interrupt the emission of ozone when the condition *C = C** occurs, where *C** is a predetermined threshold value necessary to obtain a correct sanitation of the closed environment;
      - estimate a preliminary value ${t}_{a}^{0}$ of the decomposition time necessary to pass from the condition *C* = *C** to the condition C = *C*₀ in the closed environment, where *C*₀ is a predetermined threshold value below which the concentration *C* of ozone is not harmful to people;
      - monitor the reduction of the value of concentration *C* in the closed environment following the interruption of emission of ozone;
      - calculate a value *vₐ* of the decomposition speed by the equation *vₐ* ***=* Δ*C*/Δ*t,*** where Δ*C* is the reduction of value of concentration *C* which occurs in the closed environment in a time interval Δ*t*;
      - calculate a new value *tₐ* of the decomposition time by the equation *tₐ **=*** (*C* - C*₀)/*vₐ.*

Such system therefore allows to carry out a completely automated and safe sanitization of the environment, allowing the use of the environment only when the optimal conditions have been restored, without the possibility of physical damage to users. In addition, the system allows, through an iterative process, to accurately predict the time required to bring the environment back to such optimal conditions after sanitization.

This aspect is extremely useful especially in work environments, where it is necessary to plan and automate the sanitization process, optimizing times and at the same time ensuring maximum process safety.

Furthermore, the system according to the present invention allows the same ozone abatement device to be used both to reduce ozone and to measure the concentration of ozone in the closed environment. This eliminates the need for a dedicated ozone sensor.

Furthermore, the system does not need chemical reactions to measure the ozone concentration, as most of the prior art ozone sensors do. This means that less maintenance is required compared to these sensors and, moreover, this maintenance would be performed anyway on the ozone depleting device.

Advantageously, after the ozone emission is interrupted, the control unit is able to activate the ozone abatement device to reduce the concentration of ozone in the closed environment more quickly. In this way, the system of the present invention allows to have total control over the time required to have a safe environment for people who need to access the room quickly after sanitizing. In fact, knowing the estimated time to reach the safety levels, the system allows both to precisely program the entry of people into the room and to vary the power of the ozone removal to have a predetermined abatement time.

In particular, the control unit is also arranged to insert the new value *tₐ* of the decomposition time in a database arranged to associate a plurality of decomposition times with respective environmental parameters in the presence of which the decomposition time have been calculated, said new value *tₐ* of the decomposition time being associated in the database to the environmental parameters of the closed environment.

This database, therefore, allows to collect and catalogue different types of environments, united by common environmental parameters, and to associate these types of environments with the relative decomposition times *tₐ* necessary to restore an optimal condition of the environment.

In particular, the determined environmental parameters comprise, in combination or alternatively:
- air temperature,
- air humidity;
- concentration value of gases different from ozone;
- presence of particular materials in said closed environment;
- presence of particular furniture in said closed environment.

Advantageously, the control unit is adapted to estimate the preliminary value ${t}_{a}^{0}$ of the decomposition time on the basis of decomposition times present in the database.

In particular, the sanitizing system can estimate the preliminary value ${t}_{a}^{0}$ of the decomposition time of an environment on the basis of decomposition times *tₐ* previously calculated in the same environment or on the basis of decomposition times *tₐ* referred to types of environments in which there were environmental parameters similar to the new environment. This allows speeding up the following calculus that, by the monitoring the reduction in value of concentration *C*, leads to the prediction of the new decomposition time *tₐ*.

Obviously, the more data in the database, the better the estimate of the preliminary value ${t}_{a}^{0}$ will be, speeding up the overall process of predicting the new value of *tₐ*.

Furthermore, in a possible embodiment of the invention, the system includes a plurality of sanitizing devices that work in parallel using the same database, located remotely and connected to the network, for example by sharing in the cloud. In particular, the sanitizing devices work within a mesh network.

In particular, the control unit is adapted to estimate a preliminary value ${t}_{a}^{0}$ by means of machine learning algorithms, starting from the values present on the database.

Advantageously, the sanitizing device is adapted to carry out a notification of the preliminary value ${t}_{a}^{0}$ and/or of the new value *tₐ* of the decomposition time.

In this way, sanitation operators and/or users of the environment to be sanitized can know the estimated time in order to consider the environment no longer harmful and act accordingly, without having to wait for the sensor to measure a concentration value *C = C*.*

Advantageously, the control unit is adapted to carry out an iteration of the value *vₐ* of the decomposition speed and of the new value *tₐ* of the decomposition time, said iteration ending when the new value *tₐ* of the decomposition time has a difference lower than a predetermined threshold between two successive cycles of this iteration.

This allows for a more precise approximation of the decomposition time.

In particular, when the condition *C* = *C*₀ is reached in the closed environment, a step of verification of the previously calculated decomposition time can be provided. If the real decomposition time is different from the calculated one, this real decomposition time is replaced within the database.

Advantageously, the control unit is further arranged to:
- calculate an optimal value *Q̇ₒₜₜ* of amount of ozone emitted in the unit of time to have in the closed environment a concentration of ozone equal to C = C* in a predetermined sanitizing time;
- control the generator to have an amount of ozone emitted in the unit of time *Q̇ = Q̇ₒₜₜ.*

This way, it is possible to set the time needed to sanitize the environment according to specific needs, that is to bring the environment to have an ozone concentration equal to *C* = *C**.

For example, this can allow to adjust the speed of the sanitization procedure on the basis of the decomposition time expected in that environment, so as to have homogeneity of overall times in different environments.

Furthermore, this allows to predict with greater accuracy the overall time to carry out both sanitation and abatement, increasing the autonomy and optimization of the system.

Advantageously, a diffusion device is also provided adapted to diffuse the ozone in the closed environment according to a determined direction *d* and with a determined speed *v*.

In particular, the diffusion device is a fan. Advantageously, the fan is controlled in speed by means of PWM technique.

The diffusion device allows the ozone-containing flow to be directed towards the upper part of the closed environment, so as to diffuse it throughout the entire volume of the environment before the ozone (heavier than air) precipitates to the ground.

Advantageously, the control unit is also arranged to control the diffusion device for changing the determined direction *d* and the determined diffusion speed *v*. In this way, the control unit can change the direction and speed of the flow to adapt them to different environments or to different stages of sanitation.

In particular, an argon concentration sensor and/or a carbon monoxide concentration sensor and/or a carbon dioxide concentration sensor are also provided for measuring the concentrations of these gases within the environment.

Advantageously, a temperature sensor and/or a humidity sensor is also provided. In this way, the control unit can receive the temperature and/or humidity values and assess whether the correct operating conditions for sanitization are present in the environment.

Advantageously, at least one sensor is also provided to verify that open doors and/or windows are present in the closed environment. In this way, in the event that there are doors/windows open, the control unit can command the issuance of an alarm and prevent sanitation until they are closed.

In particular, at least one sensor is also provided to verify that people and/or animals are present in the closed environment. In this way, in the event that there are people/animals, the control unit can command the emission of an alarm and prevent sanitation or appropriately regulate the concentration of ozone in the environment in order not to harm people/animals present.

Advantageously, the control unit is connected to a management device designed to allow the monitoring of system activities and/or manual intervention by an operator, locally and/or remotely.

In particular, the management device can be a computer, a smartphone, a tablet, or similar devices.

Advantageously, the control unit is connected to a network of data connections, for example a cloud, which allows the management and monitoring of the system from remote devices. In particular, this data connection network is also aimed at carrying out statistical analyses based on the data collected by the sanitizing devices of the system.

In particular, the control unit is able to process a log showing the variation of the concentration value C in the closed environment. This allows to certify the correct sanitization procedure, in compliance with the regulations in force on the subject.

### Brief description of the drawings

The invention will be now shown with the following description of some exemplary embodiments, exemplifying but not limitative, with reference to the attached drawings in which:
- Fig. 1 shows possible logic diagram of the activities carried out by the control unit during the ozone abatement step;
- Fig. 2 shows a possible logic diagram of the activities carried out by the sanitizing device during the entire sanitation operation.

### Description of some preferred exemplary embodiments

The system for sanitizing a closed environment, according to the present invention, comprises at least one generator arranged to emit ozone and a control unit arranged to operate the generator to adjust the emission of ozone.

Furthermore, the system comprises an ozone abatement device comprising at least one capacitor adapted to generate an electric field and a current sensor configured to measure an electric current intensity value passing through the capacitor.

In particular, the ozone abatement device is configured in such a way that, when a determined amount of ozone passes through the capacitor, the electric field transforms the determined amount of ozone in a determined amount of oxygen releasing a plurality of electric charges. Such electric charges produce a ion current in the capacitor having an intensity proportional to the concentration C of ozone in the closed environment.

In particular, the transformation of ozone into oxygen occurs thanks to the electric field produced by the capacitor, which appropriately orientates the ozone dipole. As known, this transformation provides that 2 ozone atoms produce 3 oxygen atoms and 2 free electrons, which produce the ionic current in the capacitor.

Advantageously, the ozone abatement device comprises a plurality of capacitors arranged in parallel.

According to the invention the electric field produced has a value larger than 30.000 V/m.

Advantageously, the rotor of each capacitor are arranged at a distance of about 9 mm.

Advantageously, the voltage present between the plates of the capacitor is about 320 V.

Advantageously, the capacitor must be fully charged before the ozone passes. Therefore, there is a voltage between the plates of the capacitor but not a passage of current.

In particular, the control unit is able to periodically acquire a ion current intensity value passing through the capacitor from the current sensor and to calculate, based on this value, a concentration value C of ozone in the closed environment.

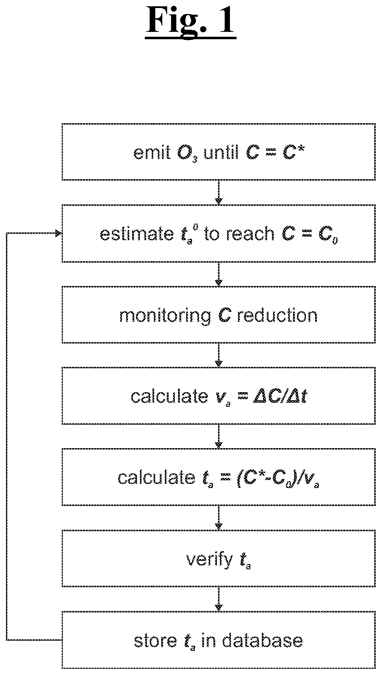

Figure 1 shows a possible logic diagram 100 of the activities carried out by the control unit to achieve the optimized and safe sanitation of the environment.

In particular, once a sanitizing device is placed inside the closed environment, the control unit is able to carry out a first step of ozone emission [101]. This emission is interrupted when an ozone concentration equal to *C* = *C** is detected inside the environment, where C* is a predetermined threshold value necessary to obtain proper sanitation of the closed environment. Alternatively, the ozone emission can be interrupted when, based on the power of the generator and the volume of the environment considered, the estimated time necessary to obtain the necessary concentration of ozone foreseen for the type of sanitization chosen for that given environment is reached.

Subsequently, the control unit estimates a preliminary value ${t}_{a}^{0}$ of the decomposition time necessary to pass from the condition *C* = *C** to the condition *C* = *C*₀ in the closed environment [102], where *C*₀ is a predetermined value of threshold below which the concentration of ozone is not harmful to people.

According to an embodiment of the invention, the control unit can interface with a database capable of associating a plurality of decomposition times with respective environmental parameters in the presence of which decomposition times have been calculated. In this way, the control unit can estimate the preliminary value ${t}_{a}^{0}$ of the decomposition time of a new environment on the basis of decomposition times *tₐ* previously calculated in the same environment or on the basis of decomposition times *tₐ* referring to types of environments in which environmental parameters similar to the new environment were present.

Once the estimate of the preliminary value ${t}_{a}^{0}$ has been made, the control unit, thanks to the current sensor, monitors the reduction of the concentration value *C* in the closed environment following the interruption of ozone emission [103], so as to be able to calculate a value *vₐ* of the decomposition speed by means of the relationship *vₐ* **=** Δ*C*/Δ*t*, where Δ*C* is the reduction of the concentration value C that occurs in the closed environment in a time interval Δ*t.*

The control unit finally proceeds to calculate a value *tₐ* of the decomposition time by the equation *tₐ =* (*C** - *C*₀)/*vₐ.*

Advantageously, the control unit is then arranged to perform an iteration of the calculation of the value *vₐ* of the decomposition speed [104] and of the new value *tₐ* of the decomposition time [105], said iteration ending when the new value *tₐ* of the decomposition time has a difference lower than a predetermined threshold between two successive cycles of this iteration.

In particular, when the condition *C* = *C*₀ is reached in the closed environment, a step can be provided of checking the previously calculated decomposition time [106].

When the new value *tₐ* is definitive, the sanitizing device is able to make a notification that signals that the environment is in a condition that is not harmful to people, allowing their eventual re-entry.

Furthermore, this new value *tₐ* is then entered into the database by associating it with the environmental parameters of the closed environment to allow an improvement in the calculation of the preliminary value ${t}_{a}^{0}$ in new environments [107].

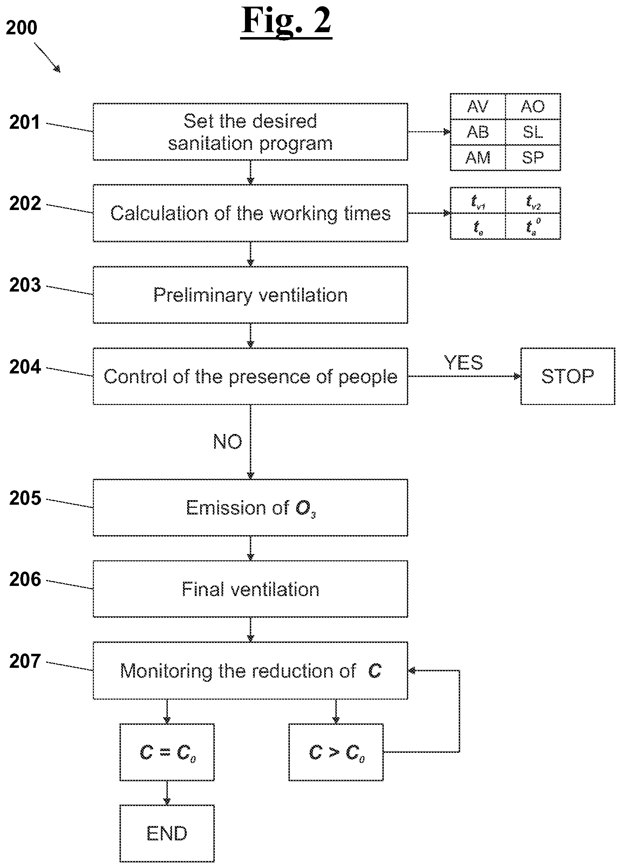

Fig. 2 shows a possible logic diagram 200 of the activities carried out by the sanitizing device during the entire sanitation operation.

In particular, a first step is provided in which the system asks the user to set the desired sanitation program [201]. The system provides for the choice between various types of sanitizations, some examples of which are given below:
- Anti-Virus (AV);
- Anti-Bacteria (AB);
- Anti Mould (AM);
- Anti-Odours (AO);
- Light Sanitation (SL);
- Heavy Sanitation (SP).

Once the sanitation program has been set, the system proceeds with the calculation of the working times [202], including:
- the time *t*_{*v*1} necessary for the preliminary ventilation of the environment before the emission of ozone;
- the time *tₒ* necessary for the ozone emission step;
- the time ${t}_{a}^{0}$ necessary for the ozone abatement step: this value, as mentioned above, is a preliminary estimate of the final value;
- the time *t*_{*v*2} necessary for the ventilation of the environment following the emission of ozone.

Subsequently, the sanitizing device proceeds to the operative steps of sanitization, starting from the preliminary ventilation [203], in particular using a fan regulated in speed by the PWM technique.

At the end of the preliminary ventilation, the system emits an alarm that indicates the start of sanitation and proceeds, using a presence sensor, to check that there are no moving bodies, such as people or animals, in the environment to be sanitized [204].

In the event that moving bodies are detected, the system interrupts the sanitation process, preventing people or animals from breathing high concentrations of ozone. If, on the other hand, no moving bodies are detected in the environment, the system proceeds with the ozone emission step [205] until an ozone concentration equal to *C* = *C** is reached, as shown in detail in the figure 1.

At the end of the ozone emission step, the sanitizing device then proceeds to a further step of ventilation of the environment to increase the dispersion of ozone [206]. The control unit can also activate the ozone abatement device to accelerate the abatement times.

Finally, the sanitizing device, through the current sensor, monitors the reduction of the ozone concentration in the environment, until a condition *C* = *C*₀ [207] is reached. Once this condition has been reached, as shown in detail in figure 1, the sanitizing device ends the sanitization process and emits an acoustic and/or visual signal that notifies the environment is habitable again.

## Claims

1. A system for sanitizing a closed environment, said closed environment being associated with determined environmental parameters, said system comprising:
- at least one generator arranged to emit ozone in said closed environment;
- a control unit arranged to control said generator to adjust the emission of ozone;
said system **characterized in that** it further comprises an ozone abatement device comprising:
- at least one capacitor arranged to generate an electric field having an intensity value greater than 30.000 V/m;
- a current sensor arranged to measure an electric current intensity value passing through said capacitor;
said ozone abatement device being configured in such a way that, when a determined amount of ozone passes through said or each capacitor, said electric field transforms said determined amount of ozone in a determined amount of oxygen releasing a plurality of electric charges, said plurality of electric charges producing a ion current in said capacitor, said ion current having an intensity proportional to the concentration *C* of ozone in said closed environment, **and in that** said control unit is arranged to:
- periodically acquire from said current sensor an intensity value of ion current passing through said capacitor;
- calculate, on the basis of said intensity value of ion current, a value of concentration *C* of ozone in said closed environment;
- command an emission of ozone by said generator and interrupt said emission of ozone when the condition *C = C** occurs, where *C** is a predetermined threshold value necessary to obtain a correct sanitation of said closed environment;
- estimate a preliminary value ${t}_{a}^{0}$ of the decomposition time necessary to pass from the condition *C = C** to the condition *C = C*₀ in said closed environment, where *C*₀ is a predetermined threshold value below which said concentration *C* of ozone is not harmful to people;
- monitor the reduction of said value of concentration *C* in said closed environment following the interruption of emission of ozone;
- calculate a value *vₐ* of the decomposition speed by the equation *vₐ =* Δ*C*/Δ*t,* where Δ*C* is the reduction of said value of concentration *C* which occurs in said closed environment in a time interval Δ*t*;
- calculate a new value *tₐ* of the decomposition time by the equation *tₐ =* (*C** - *C*₀)/*vₐ.*

2. The system for sanitizing a closed environment, according to claim 1, wherein said control unit is also arranged to insert said new value *tₐ* of the decomposition time in a database arranged to associate a plurality of decomposition times with respective environmental parameters in the presence of which said decomposition times have been calculated, said new value *tₐ* of the decomposition time being associated in said database to said environmental parameters of said closed environment.

3. The system for sanitizing a closed environment, according to claim 2, wherein said control unit is arranged to estimate said preliminary value ${t}_{a}^{0}$ of the decomposition time on the basis of decomposition times present in said database.

4. The system for sanitizing a closed environment, according to claim 1, wherein said control unit is arranged to perform an iteration of the calculation of said value *vₐ* of the decomposition speed and of said new value *tₐ* of the decomposition time, said iteration ending when said new value *tₐ* of the decomposition time results to have a difference lower than a predetermined threshold between two successive cycles of said iteration.

5. The system for sanitizing a closed environment, according to claim 2, wherein said determined environmental parameters comprise, in combination or alternatively:
- air temperature,
- air humidity;
- concentration value of gases different from ozone;
- presence of particular materials in said closed environment;
- presence of particular furniture in said closed environment.

6. The system for sanitizing a closed environment, according to claim 1, wherein said control unit is arranged to:
- calculate an optimal value *Q̇ₒₜₜ* of amount of ozone emitted in the unit of time to have in said closed environment a concentration of ozone *C = C** in a predetermined sanitizing time;
- control said generator to have an amount of ozone emitted in the unit of time *Q̇ = Q̇ₒₜₜ.*

7. The system for sanitizing a closed environment, according to claim 1, wherein a diffusion device is also provided arranged to diffuse ozone in said closed environment according to a determined direction *d* and with a determined speed *v*.

8. The system for sanitizing a closed environment, according to claim 7, wherein said control unit is further arranged to control said diffusion device to modify said determined direction *d* and said determined diffusion speed *v*.

9. The system for sanitizing a closed environment, according to any of the previous claims, wherein said control unit is connected to a management device arranged to allow the monitoring of system activities and/or a manual intervention by an operator in local and/or remotely.

10. The system for sanitizing a closed environment, according to any of the previous claims, wherein said control unit is arranged to process a log showing the variation of said value of concentration *C* in said closed environment.

## Patentansprüche

1. System zum Sterilisieren einer geschlossenen Umgebung, wobei die geschlossene Umgebung mit bestimmten Umgebungsparametern assoziiert ist, wobei das System Folgendes umfasst:
- zumindest einen Generator, der dazu angeordnet ist, Ozon in der geschlossenen Umgebung zu emittieren;
- eine Steuereinheit, die dazu angeordnet ist, den Generator zu steuern, um die Emission von Ozon einzustellen;
wobei das System **dadurch gekennzeichnet ist, dass** es ferner eine Ozonabbauvorrichtung umfasst, umfassend:
- zumindest einen Kondensator, der dazu angeordnet ist, ein elektrisches Feld mit einem Intensitätswert größer als 30.000 V/m zu erzeugen;
- einen Stromsensor, der dazu angeordnet ist, einen Wert der elektrischen Stromintensität, die durch den Kondensator verläuft, zu messen;
wobei die Ozonabbauvorrichtung auf eine solche Weise konfiguriert ist, dass, wenn eine bestimmte Menge an Ozon durch den oder jeden Kondensator verläuft, das elektrische Feld die bestimmte Menge an Ozon in eine bestimmte Menge an Sauerstoff umwandelt, die eine Vielzahl von elektrischen Ladungen freisetzt, wobei die Vielzahl von elektrischen Ladungen einen Ionenstrom in dem Kondensator produziert, wobei der Ionenstrom eine Intensität proportional zu der Konzentration **C** von Ozon in der geschlossenen Umgebung aufweist,
**und dass** die Steuereinheit zu Folgendem angeordnet ist:
- periodisches Erfassen, von dem Stromsensor, eines Intensitätswertes von Ionenstrom, der durch den Kondensator verläuft;
- Berechnen, auf der Grundlage des Intensitätswertes von Ionenstrom, eines Konzentrationswertes **C** von Ozon in der geschlossenen Umgebung;
- Befehlen einer Emission von Ozon durch den Generator und Unterbrechen der Emission von Ozon, wenn die Bedingung **C = C*** auftritt, wobei **C*** ein vorbestimmter Schwellenwert ist, der erforderlich ist, um eine korrekte Sterilisation der geschlossenen Umgebung zu erhalten;
- Schätzen eines vorläufigen Wertes ${t}_{a}^{0}$ der Zersetzungszeit, die erforderlich ist, um von der Bedingung ***C = C**** zu der Bedingung ***C = C₀*** in der geschlossenen Umgebung überzugehen, wobei ***C*₀** ein vorbestimmter Schwellenwert ist, unter dem die Konzentration **C** von Ozon nicht schädlich für Menschen ist;
- Überwachen der Reduktion des Konzentrationswertes **C** in der geschlossenen Umgebung nach der Unterbrechung von Emission von Ozon;
- Berechnen eines Wertes ***νₐ*** der Zersetzungsgeschwindigkeit durch die Gleichung ***vₐ** =* ***ΔC*/*Δt,*** wobei ***ΔC*** die Reduktion des Konzentrationswertes **C** ist, die in der geschlossenen Umgebung in einem Zeitintervall ***Δt*** auftritt;
- Berechnen eines neuen Wertes *tₐ* der Zersetzungszeit durch die Gleichung ***tₐ = (C* - C₀)*/*vₐ.***

2. System zum Sterilisieren einer geschlossenen Umgebung gemäß Anspruch 1, wobei die Steuereinheit auch dazu angeordnet ist, den neuen Wert ***tₐ*** der Zersetzungszeit in eine Datenbank einzufügen, die dazu angeordnet ist, eine Vielzahl von Zersetzungszeiten mit jeweiligen Umgebungsparametern zu assoziieren, in deren Gegenwart die Zersetzungszeiten berechnet worden sind, wobei der neue Wert ***tₐ*** der Zersetzungszeit in der Datenbank mit den Umgebungsparametern der geschlossenen Umgebung assoziiert ist.

3. System zum Sterilisieren einer geschlossenen Umgebung gemäß Anspruch 2, wobei die Steuereinheit dazu angeordnet ist, den vorläufigen Wert ${t}_{a}^{0}$ der Zersetzungszeit auf der Grundlage von Zersetzungszeiten, die in der Datenbank vorhanden sind, zu schätzen.

4. System zum Sterilisieren einer geschlossenen Umgebung gemäß Anspruch 1, wobei die Steuereinheit dazu angeordnet ist, eine Iteration der Berechnung des Wertes ***vₐ*** der Zersetzungsgeschwindigkeit und des neuen Wertes ***tₐ*** der Zersetzungszeit durchzuführen, wobei die Iteration endet, wenn der neue Wert ***tₐ*** der Zersetzungszeit darin resultiert, eine Differenz niedriger als eine vorbestimmte Schwelle zwischen zwei aufeinanderfolgenden Zyklen der Iteration aufzuweisen.

5. System zum Sterilisieren einer geschlossenen Umgebung gemäß Anspruch 2, wobei die bestimmten Umgebungsparameter in Kombination oder alternativ Folgendes umfassen:
- Lufttemperatur,
- Luftfeuchtigkeit;
- Konzentrationswert von Gasen, die sich von Ozon unterscheiden;
- Vorhandensein von bestimmten Materialien in der geschlossenen Umgebung;
- Vorhandensein von bestimmten Möbeln in der geschlossenen Umgebung.

6. System zum Sterilisieren einer geschlossenen Umgebung gemäß Anspruch 1, wobei die Steuereinheit zu Folgendem angeordnet ist:
- Berechnen eines optimalen Wertes *Q̇ₒₜₜ* der Menge an Ozon, die in der Zeiteinheit emittiert wird, um in der geschlossenen Umgebung eine Konzentration von Ozon ***C* = *C**** in einer vorbestimmten Sterilisationszeit aufzuweisen;
- Steuern des Generators, um eine Menge an Ozon aufzuweisen, die in der Zeiteinheit *Q̇ = Q̇ₒₜₜ* emittiert wird.

7. System zum Sterilisieren einer geschlossenen Umgebung gemäß Anspruch 1, wobei eine Diffusionsvorrichtung auch bereitgestellt ist, die dazu angeordnet ist, Ozon in der geschlossenen Umgebung gemäß einer bestimmten Richtung ***d*** und mit einer bestimmten Geschwindigkeit ***v*** zu diffundieren.

8. System zum Sterilisieren einer geschlossenen Umgebung gemäß Anspruch 7, wobei die Steuereinheit ferner dazu angeordnet ist, die Diffusionsvorrichtung zu steuern, um die bestimmte Richtung ***d*** und die bestimmte Diffusionsgeschwindigkeit ***v*** zu modifizieren.

9. System zum Sterilisieren einer geschlossenen Umgebung gemäß einem der vorhergehenden Ansprüche, wobei die Steuereinheit mit einer Verwaltungsvorrichtung verbunden ist, die dazu angeordnet ist, die Überwachung von Systemaktivitäten und/oder einen manuellen Eingriff durch einen Bediener lokal und/oder entfernt zu ermöglichen.

10. System zum Sterilisieren einer geschlossenen Umgebung gemäß einem der vorhergehenden Ansprüche, wobei die Steuereinheit dazu angeordnet ist, ein Protokoll zu verarbeiten, das die Variation des Konzentrationswertes ***C*** in der geschlossenen Umgebung zeigt.

## Revendications

1. Système de désinfection d'un environnement fermé, ledit environnement fermé étant associé à des paramètres environnementaux déterminés, ledit système comprenant :
- au moins un générateur conçu pour émettre de l'ozone dans ledit environnement fermé ;
- une unité de commande conçue pour commander audit générateur d'ajuster l'émission d'ozone ;
ledit système **étant caractérisé en ce qu'il** comprend en outre un dispositif de diminution de l'ozone comprenant :
- au moins un condensateur conçu pour générer un champ électrique ayant une valeur d'intensité supérieure à 30 000 V/m ;
- un capteur de courant agencé pour mesurer une valeur d'intensité de courant électrique traversant ledit condensateur ;
ledit dispositif de diminution de l'ozone étant configuré de telle sorte que, lorsqu'une quantité déterminée d'ozone traverse ledit ou chaque condensateur, ledit champ électrique transforme ladite quantité déterminée d'ozone en une quantité déterminée d'oxygène libérant une pluralité de charges électriques, ladite pluralité de charges électriques produisant un courant ionique dans ledit condensateur, ledit courant ionique ayant une intensité proportionnelle à la concentration *C* d'ozone dans ledit environnement fermé,
**et en ce que** ladite unité de commande est conçue pour :
- acquérir périodiquement à partir dudit capteur de courant une valeur d'intensité du courant ionique traversant ledit condensateur ;
- calculer, sur la base de ladite valeur d'intensité du courant ionique, une valeur de concentration *C* d'ozone dans ledit environnement fermé ;
- commander une émission d'ozone par ledit générateur et interrompre ladite émission d'ozone lorsque la condition *C = C** se produit, où C* est une valeur seuil prédéterminée nécessaire pour obtenir un assainissement correct dudit environnement fermé ;
- estimer une valeur préliminaire ${t}_{a}^{0}$ du temps de décomposition nécessaire pour passer de la condition *C = C** à la condition *C* = *C*₀ dans ledit environnement fermé, où *C*₀ est une valeur seuil prédéterminée en dessous de laquelle ladite concentration *C* d'ozone n'est pas nocive pour les personnes ;
- surveiller la réduction de ladite valeur de concentration *C* dans ledit environnement fermé suite à l'interruption de l'émission d'ozone ;
- calculer une valeur *vₐ* de la vitesse de décomposition par l'équation *vₐ* = Δ*C*/Δ*t,* où Δ*C* est la réduction de ladite valeur de concentration *C* qui se produit dans ledit environnement fermé dans un intervalle de temps Δ*t* ;
- calculer une nouvelle valeur *tₐ* du temps de décomposition par l'équation *tₐ =* (*C** - *C*₀)/*vₐ.*

2. Système de désinfection d'un environnement fermé, selon la revendication 1, dans lequel ladite unité de commande est également agencée pour insérer ladite nouvelle valeur *tₐ* du temps de décomposition dans une base de données conçue pour associer une pluralité de temps de décomposition à des paramètres environnementaux respectifs en présence desquels lesdits temps de décomposition ont été calculés, ladite nouvelle valeur *tₐ* du temps de décomposition étant associée dans ladite base de données auxdits paramètres environnementaux dudit environnement fermé.

3. Système de désinfection d'un environnement fermé, selon la revendication 2, dans lequel ladite unité de commande est conçue pour estimer ladite valeur préliminaire ${t}_{a}^{0}$ du temps de décomposition sur la base des temps de décomposition présents dans ladite base de données.

4. Système de désinfection d'un environnement fermé, selon la revendication 1, dans lequel ladite unité de commande est conçue pour effectuer une itération du calcul de ladite valeur *vₐ* de la vitesse de décomposition et de ladite nouvelle valeur *tₐ* du temps de décomposition, ladite itération se terminant lorsque ladite nouvelle valeur *tₐ* du temps de décomposition résulte en une différence inférieure à un seuil prédéterminé entre deux cycles successifs de ladite itération.

5. Système de désinfection d'un environnement fermé, selon la revendication 2, dans lequel lesdits paramètres environnementaux déterminés comprennent, en combinaison ou en variante :
- température de l'air,
- humidité de l'air ;
- valeur de concentration des gaz différents de l'ozone ;
- présence de matériaux particuliers dans ledit environnement fermé ;
- présence de meubles particuliers dans ledit environnement fermé.

6. Système de désinfection d'un environnement fermé, selon la revendication 1, dans lequel ladite unité de commande est conçue pour :
- calculer une valeur optimale *Q̇ₒₜₜ* de la quantité d'ozone émise dans l'unité de temps pour avoir dans ledit environnement fermé une concentration d'ozone *C = C** dans un temps de désinfection prédéterminé ;
- commander ledit générateur pour qu'une quantité d'ozone soit émise dans l'unité de temps *Q̇* = *Q̇ₒₜₜ.*

7. Système de désinfection d'un environnement fermé, selon la revendication 1, dans lequel un dispositif de diffusion est également prévu agencé pour diffuser de l'ozone dans ledit environnement fermé selon une direction déterminée d et avec une vitesse déterminée *v*.

8. Système de désinfection d'un environnement fermé, selon la revendication 7, dans lequel ladite unité de commande est en outre conçue pour commander ledit dispositif de diffusion afin de modifier ladite direction déterminée d et ladite vitesse de diffusion déterminée *v*.

9. Système de désinfection d'un environnement fermé, selon l'une quelconque des revendications précédentes, dans lequel ladite unité de commande est connectée à un dispositif de gestion conçu pour permettre la surveillance des activités du système et/ou une intervention manuelle d'un opérateur en local et/ou à distance.

10. Système de désinfection d'un environnement fermé, selon l'une quelconque des revendications précédentes, dans lequel ladite unité de commande est conçue pour traiter un journal montrant la variation de ladite valeur de concentration C dans ledit environnement fermé.
